# EUROPEAN PATENT SPECIFICATION

(11) **EP 3 283 022 B1**
(45) Date of publication and mention of the grant of the patent: **05.06.2019**
(21) Application number: 16712409.8
(22) Date of filing: 18.03.2016
(51) Int. Cl.: A61F 6/00, A61F 6/04

(54) **A CONDOM HOLDER WHICH MAY BE INTEGRATED DIRECTLY INTO A CONDOM WRAPPER TO ASSIST USERS IN THE CORRECT APPLICATION OF A CONDOM**
KONDOMHALTER ZUR DIREKTEN INTEGRATION IN EINE KONDOMVERPACKUNG ZUR UNTERSTÜTZUNG VON BENUTZERN BEI DER KORREKTEN ANWENDUNG EINES KONDOMS
SUPPORT DE PRÉSERVATIF QUI PEUT ÊTRE DIRECTEMENT INTÉGRÉ DANS UN EMBALLAGE DE PRÉSERVATIF AFIN D'AIDER LES UTILISATEURS DANS L'APPLICATION CORRECTE D'UN PRÉSERVATIF

(30) Priority: 13.04.2015 GB 201506239
(43) Date of publication of application: 21.02.2018
(73) Proprietor: Hore, Michael, Hazlemere, Buckinghamshire HP15 7JJ (GB)
(72) Inventor: Hore, Michael, Hazlemere, Buckinghamshire HP15 7JJ (GB)
(74) Representative: Dehns
(86) International application number: PCT/GB2016/050762
(87) International publication number: WO 2016/166506

(56) References cited:
- WO-A2-2008/061522
- CA-A1- 2 541 878
- DE-A1- 3 237 566

## Description

The present invention relates to a condom holder for holding a condom, and to a method of packaging a condom. In an example the condom holder is for holding the condom within a wrapper.

Condoms are a widely used and commonly known form of contraception. There are various standards that relate to condoms and set requirements for their manufacture and their performance, in particular ISO4074 & ISO23409. When properly used a condom is a highly effective form of contraception and, contrary to popular believe, failures of the condom resulting in unwanted pregnancies or exposure to sexually transmitted diseases are rare. In fact it is considered that incorrect use of the condom is a bigger cause of unwanted pregnancies or exposure to sexually transmitted diseases than failure of the condom. As a result, a problem exists that arises from misuse of condoms, such as may be caused by the user's inexperience, over confidence or lack of knowledge. This misuse frequently occurs during the application of the condom as this is often done in a rush and while the user is distracted. In many cases the user may be unaware that the misuse has even occurred.

Common mistakes in the application of condoms include:
- incorrectly orientating the condom and touching the outer surface against the tip of the penis first and then 'flipping' it over to the correct side before unrolling it;
- trapping air in the reservoir tip of the condom, leaving no space to contain the semen; and
- transferring bodily fluids or non-water-based lubricants (which may damage latex condoms) to the outer surface of the condom.

Thus, it will be appreciated that there is a need for a way to reduce the possibility of misuse of condoms.

CA 2541878 discloses a package for a contraceptive device. DE 3237566 discloses a package for a condom.

According to a first aspect, the present invention provides a holder for a condom as set out in claim 1.

The holder may for example be a part of a packaging system for the condom, and preferably the condom is attached to the holder when it is sealed within its packaging. Thus, the condom may be provided to a user in a form that promotes correct use. By ensuring that the outer surface of the condom is fully covered while the holder is still holding the condom then it becomes possible to reduce or even fully avoid the risk of contamination of the outer surface of the condom, particularly from contact with hands, body oils, or a penis, all of
which can reduce the effectiveness of the condom at preventing pregnancy or transmission of sexually transmitted diseases. Moreover, by pinching the tip of the condom whilst the condom is held in the holder then the holder promotes correct application of the condom reducing or fully avoiding the risk of air being left within the tip of the condom after it has been applied to the penis. The fact that the tip of the condom is gripped by pinching within the closable pocket also acts to securely attach the condom to the holder. Advantageously, the holder is arranged to continue to pinch the tip and shield the outer surface of the condom until after the user has started to unroll the condom and the condom has therefore had the opportunity to seal around the head of the penis thus preventing air from entering the tip.

Preferably, the holder comprises a first strip having a first end and a second end; and a second strip having a first end and a second end; wherein the first end of the first strip is joined to the second strip at a point distant from the first end of the second strip, and the first end of the second strip is joined to the first strip at a point distant from the first end of the first strip, whereby the closable pocket is formed between the first strip and the second strip. Thus, the closable pocket may be formed by a loop of material made from a part of the first strip extending from the first end of the first strip to the point at which the first end of the second strip joins the first strip and from a part of the second strip extending from the first end of the second strip to the point at which the first end of the first strip joins the second strip. The joins between the strips may use adhesive elements, or heat sealing, for example.

Preferably the first strip has an opening adjacent to the closable pocket for receiving the tip of the condom, with the strips being arranged to pinch the tip in the closed pocket when the tip is passed through the opening. The opening may be a hole in the first strip, such as a circular hole or a notch extending from the first end of the strip.

Alternatively, the holder comprises a first strip having a first end and a second end; and a second strip having a first end and a second end; wherein the first end of the second strip is sealed to the first strip at a first point distant from both ends of the first strip, and the second end of the second strip is sealed to the first strip at a second point distant from both ends of the first strip, such that the closable pocket is formed between the second strip and first strip.

The holder comprises a hole in the first strip located between the first and second points and opening into the closeable pocket; and a line of perforations in the first strip, said line extending across the width of the first strip and intersecting the hole.

The second strip may be arranged to provide the shield region for completely covering the outer surface of the condom when the condom is, in use, mounted in the holder with the tip in the closable pocket. Thus, by means of two strips it is possible to form a holder that pinches the tip of the condom, holds the condom, and shields the outer surface of the condom.

The first and second strips may be connected, for example via a seal such as a heat seal, or by adhesive elements to an outer wrapper that encloses both the holder and the rolled condom. The wrapper may have the same basic function as a traditional condom wrapper and hence it may enclose the condom for storage, for transport and for sale of the condom, for example. With the preferred arrangement having the strips connected to the wrapper, when the user unwraps the wrapper, the holder and condom advantageously remain attached to the wrapper or to portions of the wrapper and cannot fall out of the wrapper.

A portion of one or both strips of the holder may be folded into a Z-shaped configuration, preferably a portion that extends away from the closable pocket and shield region. This enables a relatively long strip to be held within a relatively short wrapper. In some examples the strips are first folded about a midpoint, which might be a centre of the strips, and then folded into a Z-shape. This means that the length of strip held within the width of the Z-fold is six times the width of the Z-fold. This Z-shaped configuration may be made straight by pulling on ends of the two strips to unfold the Z. Even when the Z-folds are pulled straight the shield region still shields the outer surface of the condom, protecting it from contamination, while the closable pocket holds the condom by the tip.

The wrapper may be opened by tearing, for example to separate the wrapper into two portions each attached to an end of one of the strips. In one preferred example the wrapper is opened by removing a central tear strip from about the wrapper. Pulling on the two portions of the outer wrapper will pull on the ends of the strips and hence unfold the Z-shapes, if present, as described above.

Alternatively, the holder may be packaged in a conventional outer wrapper for a condom, such as those being formed by two separate heat sealable sheets to form a top and bottom layer and heat sealed on all four edges. In this example, the strips would not be connected directly to the outer wrapper and the user must open the condom wrapper as normal, remove the condom held in the holder and then pull directly on the ends of the Z-shaped portions to unroll the condom.

It is preferred for the strips to be joined to form the closable pocket by means of releasable couplings, for example via couplings that may be pulled apart under sufficient tension in order to open the closable pocket, preferably by releasing the first ends of each strip from the other strip. The couplings may be unpeelable couplings, for example formed by adhesive elements or by heat sealing. The pulling action, used to open the closable pocket, acts upon the inside surfaces of the two strips that form the closable pocket, forcing them to come tightly together, completely forcing any air out of the tip that may have become trapped. The couplings may be used in combination with the Z-folds in which case they are preferably arranged to release in response to a tensile force larger than that required to unfold the Z-folds. This means that the Z-folds will unfold first, which allows the user to place the condom, and then the strips will release from one another, breaking the pocket open and releasing the condom tip, after which the condom may be unrolled.

In a preferred example, a free end of the first or second strip includes an unrolling strap that is, in use, rolled up along the outer surface of the rolled condom. Thus, the unrolling strap may be rolled up with the rolled part of the condom when the condom is held in the holder. A free end of the first or second strip may comprise an unrolling strap that is rolled up with the condom on the opposite side of the condom from the first strip's unrolling strap. With this arrangement, when the user pulls on one or both of the unrolling straps, for example by pulling on the strips, then the condom is unrolled without the user having to touch the outer surface of the condom with potentially contaminated hands.

Alternatively, the unrolling straps may be formed separately from the first and second strips. These separately formed unrolling straps may then be heat-sealed to the first and second strips. Preferably, the separately formed unrolling straps are sealed to the first and/or second strips after the first and second strips have been joined to form the closable pocket. Alternatively, the separately formed unrolling straps may both be heat sealed to the first strip in an example where the first strip extends beyond either side of the closable pocket. In this example, each of the unrolling straps is sealed to a region of the first strip near to the closable pocket.

Preferably the unrolling straps are arranged so that the condom will begin to unroll when tension is applied to the first and second strips, and if this tension continues to increase then the first and second strips begin to pull apart, whilst the condom is unrolled slightly further, then the first and second strips fully separate from one another, for example as discussed above, whereafter the unrolling strap(s) are able to fully unroll the condom, which has been released from the pocket. This enables the holder to continue to pinch the tip and shield the outer surface of the condom until after the user has started to unroll the condom and the condom has therefore had the opportunity to substantially seal around the head of the penis thus preventing air from entering the tip. The condom is then fully unrolled only after it is safely attached to the head of the penis.

The unrolling straps may be arranged to release from the condom or to break in order to fully disconnect the condom from the holder after it has been unrolled onto a penis or the like. The entire operation can thus be achieved with no contact between the hands and the condom.

A second aspect of the invention provides a method of packaging a condom as set out in claim 13.

The holder may for example be a part of a packaging system for the condom, and preferably the condom is attached to the holder when it is sealed within its packaging. Thus, the method may include coupling the condom to the holder and subsequently sealing the condom within packaging, for example within a wrapper. The packaging for the condom may be provided with the features described above in connection with the first aspect.

Preferably, the holder comprises a first strip and a second strip and the method includes joining a first end of the first strip to the second strip at a point distant from a first end of the second strip, and joining the first end of the second strip to the first strip at a point distant from the first end of the first strip in order to form the closable pocket between the first strip and the second strip. The strips and the closable pocket may be provided with features as described above.

The first strip may be provided with an opening adjacent the closable pocket and the method may include inserting the tip of the condom through the opening into the pocket with the pocket open, and then closing the pocket to pinch the tip and thereby hold the condom. The opening may be as described above.

Alternatively, in an example wherein the holder comprises a first strip having a first end and a second end and a second strip having a first end and a second end, the method may includes joining a first end of the second strip to the first strip at a first point distant from both ends of the first strip, and joining the second end of the second strip to the first strip at a second point distant from both ends of the first strip, to form the closable pocket between the first strip and the second strip.

The second strip may be arranged to provide the shield region. Thus, the method may include completely covering the outer surface of the condom using a part of the second strip. Preferably the second strip provides the shield region and also the first strip has an opening for the tip of the condom. In this case the method includes covering the outer surface of the condom when the tip is inserted into the opening and the pocket is closed.

The method may include connecting a free end of the first or second strips to an outer wrapper, and connecting another free end of the first or second strips to a second portion of the outer wrapper, wherein the outer wrapper is for enclosing both the holder and the rolled condom. The wrapper and the connection may be as described above. The connecting step may occur before the wrapper is fully formed and/or before the wrapper is sealed. Preferably the first strip is connected to a different portion of the wrapper to the second strip.

In one example, the method includes folding a portion of one or both strips of the holder into a Z-shaped configuration. Preferably this is a portion that extends away from parts of the strips that form the closable pocket. The Z-shaped portion may be as described above.

The wrapper may be openable by tearing, for example to separate the wrapper into the first and second portions each attached to an end of one of the strips. Thus, the method may include forming the wrapper with an opening mechanism for unsealing the wrapper and separating it into two portions. A central tear strip may be provided. Alternatively, the holder may be packaged in a conventional outer wrapper for a condom, such as those being formed by two separate heat sealable sheets to form a top and bottom layer and heat sealed on all four edges. In this example the strips would not be connected directly to the outer wrapper and the user must open the condom wrapper as normal, remove the condom held in the holder and then pull directly on the ends of the Z-shaped portions to unroll the condom.

As discussed above, it is preferred for the strips to be joined to form the closable pocket by means of releasable couplings, for example via couplings that may be pulled apart under sufficient tension in order to open the closable pocket, preferably by releasing the first ends of the each strip from the other strip. The method may hence include releasably joining the strips via the first ends of the strips.

The method may include providing a second end of the first strip with an unrolling strap that is, in use, rolled up along the outer surface of the rolled condom. Thus, the method may include rolling the condom together with the unrolling strap. This could be done before or after the condom tip is pinched in the closable pocket. A second end of the second strip may be provided with an unrolling strap in a similar way, with this unrolling strap being rolled up with the condom on the opposite side of the condom from the first strip's unrolling strap.

Alternatively, the method may include attaching a first unrolling strap to the first or second strips. In this example, the method may preferably comprise attaching a second unrolling strap to the first or second strips, wherein the second unrolling strap is attached to the strip on an opposite side of the closable pocket from the first unrolling strap. The unrolling straps may be provided with features as discussed above, and in particular may be arranged to begin to unroll the condom before the first and second strips are fully separated from one another.

In a preferred method, the holder is formed using the two strips, and the condom is secured to the holder with the condom tip pinched in the closable pocket, then the holder is inserted into a wrapper blank, which is then sealed about the holder simultaneously with coupling two portions of the wrapper to the strips. Advantageously the wrapper may be sealed and attached to the strips by heat sealing.

Advantageously, the holder and methods discussed above may be used with standard condoms, and require no adjustment to the condom itself. Only the packaging is changed.

Certain preferred examples of the present invention will now be described in greater detail by way of example only and with reference to the accompanying drawings, in which:
Figure 1 shows a roll of sheet for forming into a sleeve for enclosing a condom and a die for cutting the sheet;
Figure 2 shows a cut length of the sheet with a pull tab;
Figures 3A-D show the length of sheet being formed around a former and sealed to itself to form an outer sleeve;
Figure 4 shows a strip mechanism that holds the condom being inserted into the outer sleeve;
Figure 5 shows a second sealing step whereby the strip mechanism is sealed to the outer sleeve;
Figure 6 shows an external view of the finished product from the top (upper drawing) and bottom (lower drawing) sides;
Figure 7 shows a cross-section of the strip mechanism with an unrolled condom attached;
Figure 8 shows perspectives from both above (upper drawing) and below (lower drawing) an end of each of two strips of the strip mechanism;
Figure 9 shows the two strips in an unfolded state that form the strip mechanism;
Figure 10 shows alternative shapes for an the unrolling strap that is to be rolled up with the rolled condom;
Figure 11 shows a perforated tab being unwound from the sleeve and removed therefrom, splitting the sleeve into two portions;
Figure 12 shows top (upper drawing) and bottom (lower drawing) views of the strip mechanism when the two portions of the sleeve have been pulled apart;
Figure 13 shows three steps, from left to right, in a method of applying the condom to a penis;
Figure 14 shows two side views from the right (upper drawing) and from the left (lower drawing) of the sleeve without the unrolling straps or a condom present;
Figure 15 shows opened (upper drawing) and closed (lower drawing) configurations of the strip mechanism with a condom installed thereupon;
Figure 16 shows one portion of the strip mechanism with an unrolled condom installed and the unrolling straps extending along the outer surface of the condom; and
Figure 17 shows the initial stages of unrolling the condom, in particular showing the pinched tip of the condom while the condom first begins to be unrolled onto a penis;
Figure 18 shows an alternative arrangement for the first strip with a separately formed unrolling strap being attached;
Figure 19 shows the separately formed unrolling straps extending along an unrolled condom;
Figure 20 shows the first strip of Figure 18 with a hole-and-channel at one end;
Figure 21 shows the rolled condom, with unrolling straps installed, being inserted into the closable pocket;
Figure 22 shows the rolled condom having been inserted into the closable pocket and the separately formed unrolling straps sealed to the first and second strips;
Figure 23 shows stages of sealing the first strip of Figure 18 to the second strip, which has a similar end design to the second strip of Figure 8;
Figure 24 shows an alternative arrangement for the first and second strips before attachment to one another;
Figure 25 shows the first and second strips of Figure 24 after being attached together;
Figure 26 shows the arrangement of Figures 24 and 25 when the first strip is broken during use of the mechanism; and
Figure 27 shows an alternative design of perforations for aiding removal of the central tear strip from the wrapper.

First the finished wrapped condom will be described. Later an example method of use of the wrapped condom will be described and then an example method of manufacture.

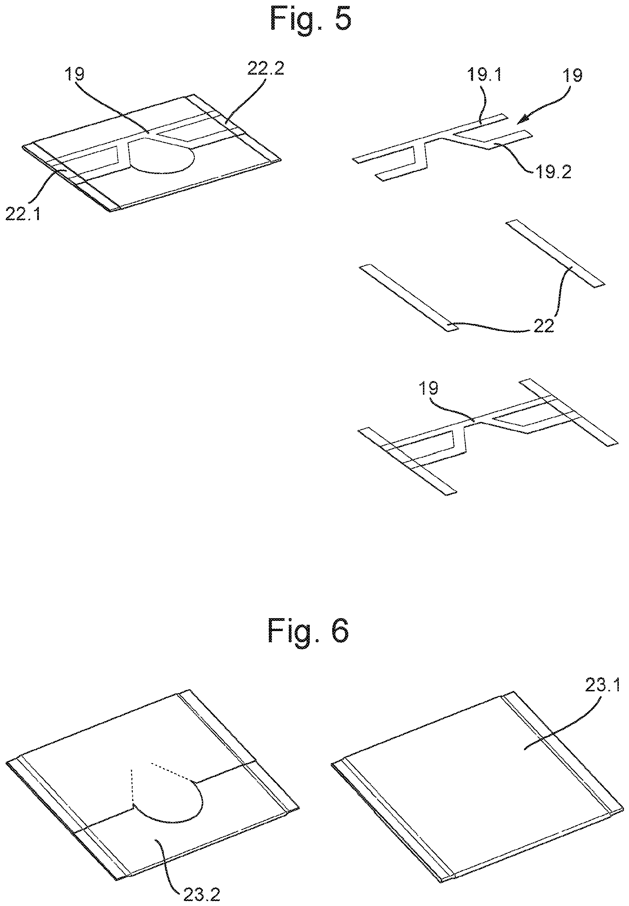

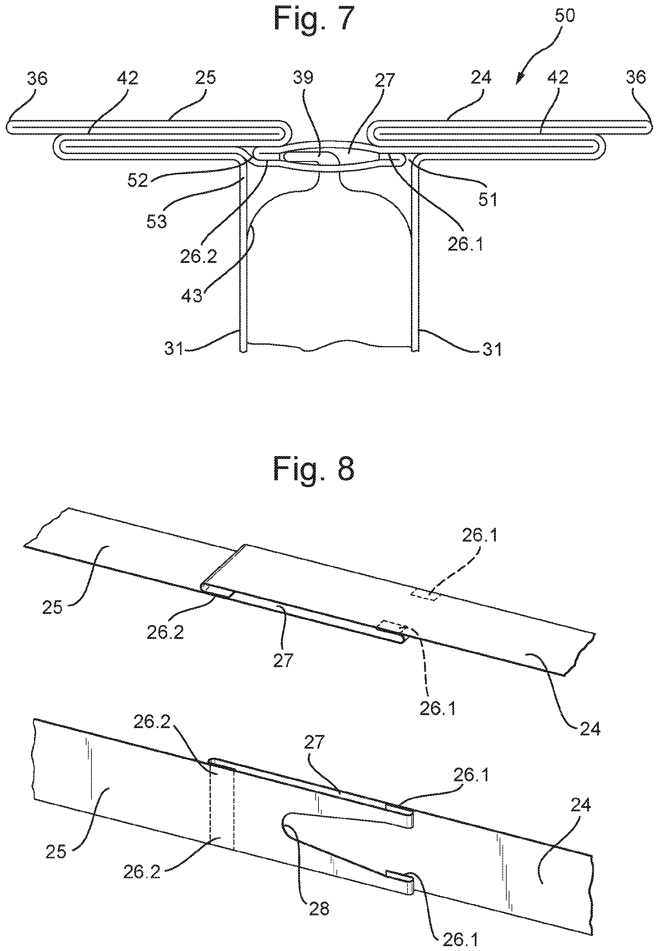

Figure 6 shows the finished, sealed, wrapped condom product from both above (upper drawing) and below (lower drawing). Before opening the product, only the outer the sleeve 23 is visible. The top surface 23.2 of the sleeve 23 has a tactile finish due to the heat seals 19.1, 19.2, 22. As described later, heat seals 19.1, 19.2 (together, 19) that seal the opposite ends 17, 18 of the sleeve blank 10 to one another, to form the sleeve 23, may be performed sequentially or in one step, but both seals 19.1 and 19.2 should be completed before the heat seals 22.1, 22.2 (together 22) are made. The sleeve 23 is sealed and formed into the wrapper 23 for the condom and holder. The heat seals 22 seal the sleeve 23 to the strips 25, 24, of the strip mechanism 50 (see Figure 7), described below, that is then stored within the wrapper 23 in the finished product. The heat seals 22.1, 22.2 seal the sides of the sleeve 23 together with the strip mechanism 50 sandwiched therebetween.

A tactile finish for the top surface of the sleeve 23 is provided by a ridge where the end 18 overlays the opposite end 17 of the sleeve 23, as well as by a pull tab 13. The bottom surface 23.1 of the sleeve 23 may be smooth. Having one surface 23.2 with a tactile finish and the other side 23.1 with a smooth finish allows the easy orientation of the finished product, even in the dark, and even by an inexperienced user.

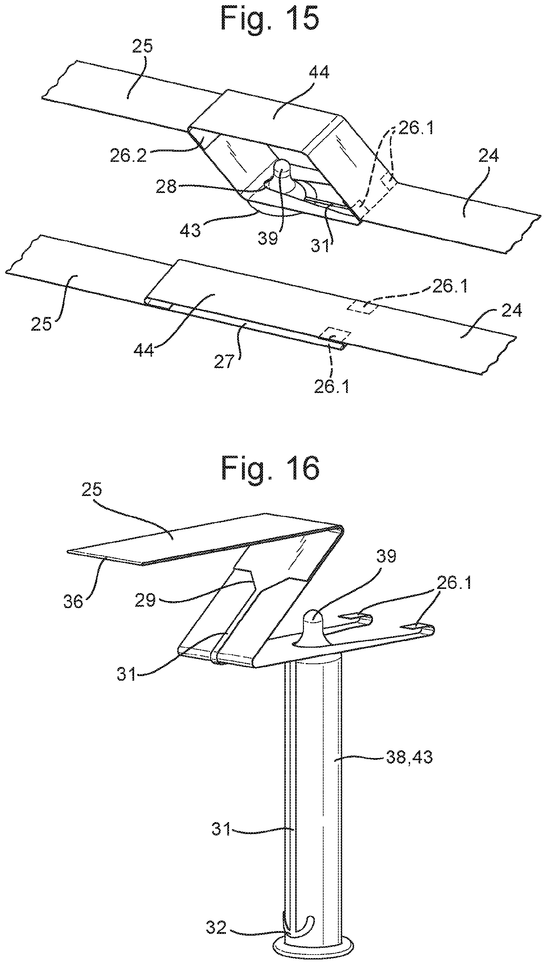

In the finished product, the strip mechanism 50 (shown in cross-section with an unrolled condom in Figure. 7) and a rolled condom 43 are stored inside the wrapper formed by the sealed sleeve 23. The strip mechanism comprises the first strip 25 and the second strip 24 that are made from a flat, heat sealable and flexible material, and that may be folded in several places to enable them to form a 'Z' shape 42. ('Z' shape may be seen closed in Fig 7 but partially opened in Fig 16) Figure 8 shows how the first strip 25 and second strip 24 are positioned so as to overlap each other and are then sealed to each other at the heat seals 26.1, 26.2. In the arrangement of these Figures, the first strip 25 has a V-shaped section 28 located at one end of the strip, where the "V" points substantially down the centreline of the first strip 25. The two ends of the "V" are folded back on themselves towards the second strip 24 and the single layer of folded back material is heat sealed to the second strip 24 at a point 26.1. Similarly, one end of the second strip 24 is folded back on itself towards the first strip 25 and the single layer of folded back material is heat sealed to the first strip 25 at a point 26.2 that is located beyond the V-shaped section of the first strip 25.

Thus, a closable pocket 27 is formed between the overlapping portions of the first and second strips 25, 24. As described in more detail below, in the finished condom wrapper product, the tip of the condom 43 sits in this pocket 27, abutting the vertex of the V-shaped section 28. The condom tip 39 is then bent to one side to lay flat against the first strip, pointing along the same direction as the "V", and the tip 39 is pinched between the first and second strips 25, 24 when the pocket 27 is closed. The tip 39 of the condom 43 is held in the pocket 27 while the rest of the rolled condom 43 sits outside the pocket 27. In the finished product, the inside surface of the rolled condom 43 abuts the sleeve 23 and the outer surface 38 of the rolled condom 43 variously abuts the V-shaped section of the first strip 25 and the second strip 24.

The aforementioned folded back portions of the strips ensure that when the first and second strips 25, 24 are pulled apart, then the area of the seal 26 between them will gradually shear apart in a controlled manner instead of suddenly failing.

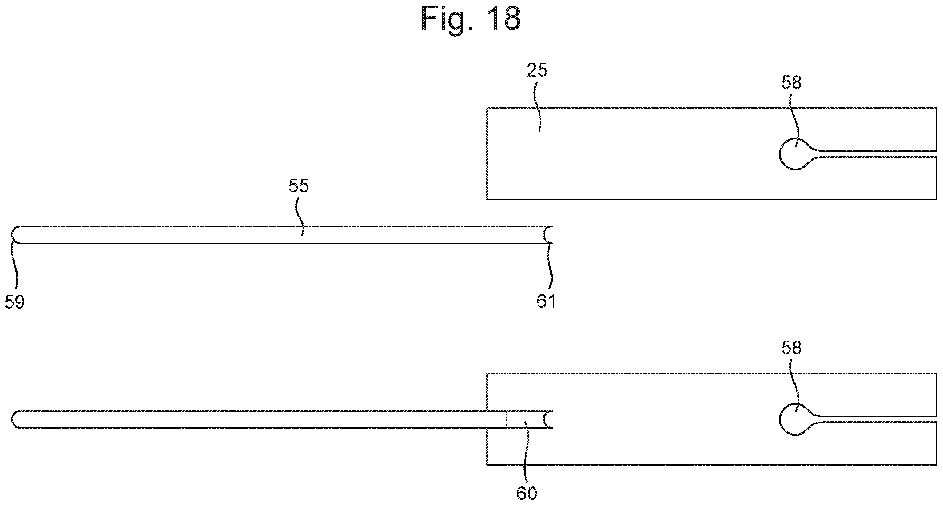

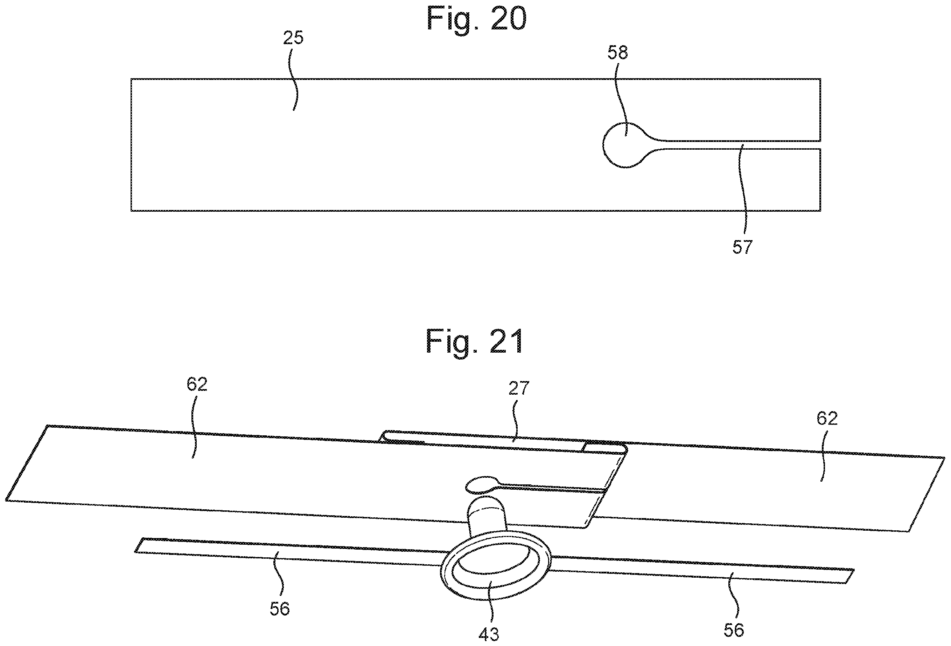

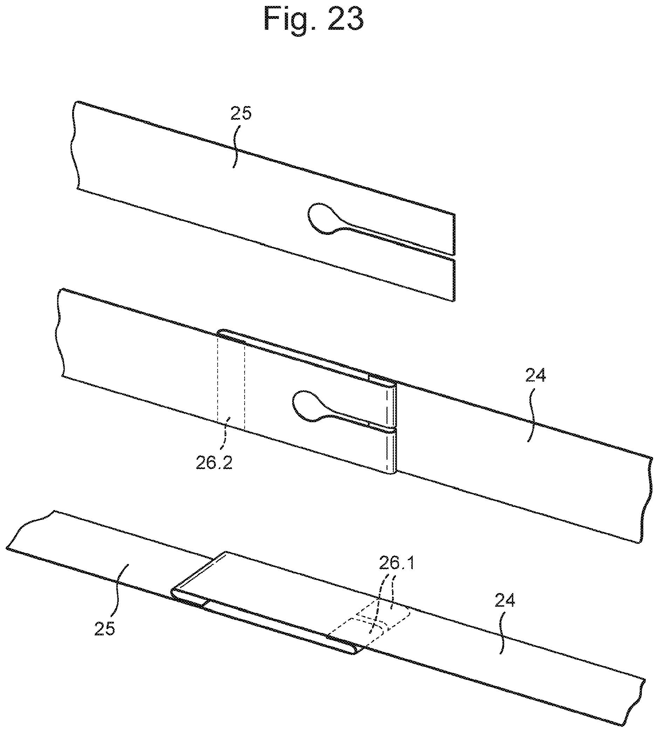

In an alternative arrangement, as shown in Figures 18 and 20-23, instead of the V-shaped section 28 described above, the first strip has a hole 58 located near one end of the strip. A channel 57 is cut out from the strip that connects the hole 58 to the end of the first strip. The hole is sized so as to receive the tip of the condom 43. Figure 23 shows how the first 25 and second 24 strips of this arrangement are joined with heat seals 26.1, 26.2. The second strip 24 of Figures 21-23 is similar to that describe above in relation to Figure 8 amongst other Figures, but it has separate unrolling straps 55/56 as discussed further below.

Aside from the above-mentioned V-shaped section 28 (or hole 58 and channel 57) of the first strip 25, the first and second strips 25, 24 are substantially identical. The following section will describe the first strip 25 but it is to be understood that all described features will be also be present for the equivalent parts of the second strip 24 as well.

The first strip 25 is folded over at a mid-point 36, producing a double-layer of strip material. Mid-point 36 is not necessarily the half-way point along each strip 24, 25, but is a point distant from each end of each the strip 24, 25. A portion of this double layer is then folded into a Z-shaped cross section 42, as shown in Figure 7. One end of the Z-shape portion is the mid-point 36, the other end of the Z-shape occurs where the two layers diverge at a divergence point 51. The divergence point 51 of each folded strip is substantially adjacent to the point 26 where that strip is sealed to the other.

At the divergence point 51, the double-layer splits into two single layers, 52 and 53. One of these single layers, 52, overlaps with the other strip and is heat-sealed thereto, in the manned described above. On the first strip 25, layer 52 contains the aforementioned V-shaped section 28 and is sealed to the second strip 24 in the manner hereinbefore described. The other of these single layers, 53, extends toward the outer surface of the condom 43, and is formed into an unrolling strap 31 that is rolled up within the condom 43 when said condom 43 is in its rolled-up state. Here "outer surface of the condom" refers to the outer surface of the condom 43 when the condom has been unrolled in normal use; the inner surface of the condom 43 being the surface that contacts a penis 45 when in normal use. It is envisaged that the condom 43 could potentially be applied to something other than a penis 45, such as a sex-toy, and references hereafter to penis 45 should not be considered as limiting.

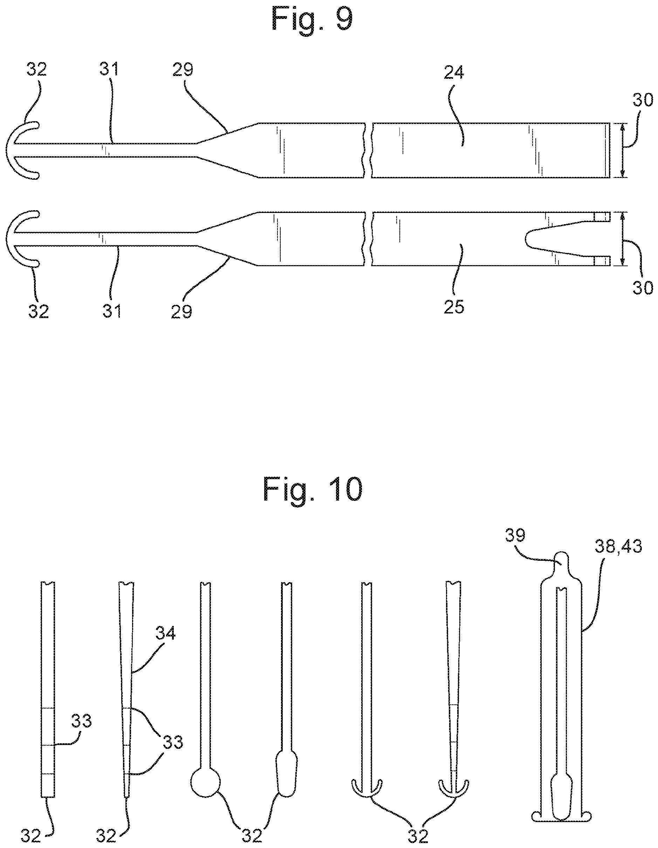

As shown in Figure 9, the first strip 25 has a width that varies along the length of the strip. The portion of each strip 24, 25, that extends from the heat seal 26.1, 26.2, respectively, to that strip's mid-point 39, has a constant first width 30 that is greater than the diameter of the rolled condom 43. At a point 29 beyond the mid-point 36 of the first strip 25, away from the heat seal 26 region, the width of the strip 25 may reduce, either as a taper or as a step-change. Beyond this taper 29, the remaining length of each strip 25 forms the unrolling strap 31. The unrolling strap 31 extends along the outer surface 38 of the unrolled condom 43, as shown in the right hand illustration in Figure 10, and is substantially rolled up within the condom 43 when rolled up.

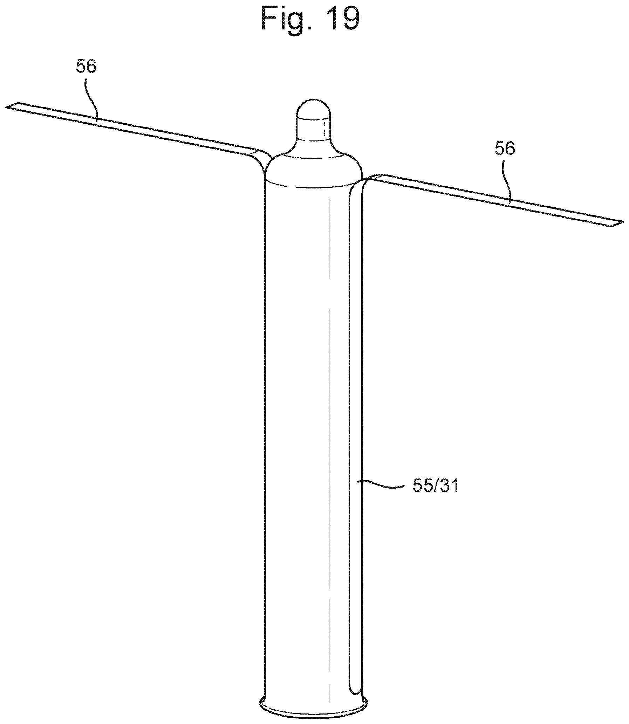

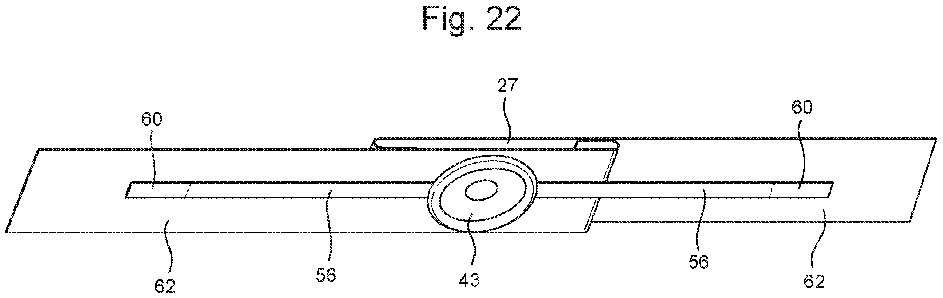

The unrolling straps 31 may alternatively be manufactured as separate pieces from the first and second strips 25, 24. These separate unrolling straps 55 are shown in Figures 18 and 19. They have an unrolling portion 55 that is rolled up with the condom, and a protruding portion 56 extending from the unrolling portion 55 toward an end 61. The protruding portion 56 allows the straps 55 to be fixed to the first strip 25 or the second strip 24 so that the straps 55 can be unrolled by a force on the strips 24, 25. The separate unrolling straps 55 may be sealed at the end 61 to a mid-point of the first or second strips 25, 24 to form a heat-seal 60. Thereafter, these unrolling straps 55 function in the same way as the unrolling straps 31 shown in Figure 9, as described below. These separately formed unrolling straps 55 are suitable for use with the any arrangements of the first 25, 65 and/or second 24, 64 strips described herein. Figure 21 shows the unrolling straps 56 already rolled up into a condom 43; in this Figure the rolled condom 43 is about to be inserted into the closable pocket 27 and the protruding portions 56 of the unrolling straps 55 are ready to be sealed to the first and second strips 25, 24 Figure 22 shows the arrangement of Figure 21 after the unrolling straps have been heat-sealed 60 to the first and second strips 24, 25 at a region 62 of each strip near the pocket 27.

The purpose of each unrolling strap 31, 55 is to unroll the condom 43 down the length of the user's penis 45 when the unrolling straps 31, 55 of the first and the second strips 25, 24 are pulled apart. As shown in Figure 10, an end 32 of the unrolling strap 31 that is distant from the taper (or step change) 29 may have a variety of cross-sectional shapes. By way of non-limiting examples, the shapes may be flat, circular, semi-circular, or hooked. The end 32 of the unrolling strap 31 is preferably shaped so as to anchor the unrolling strap 31 into the rolled condom 43 so that the unrolling strap 31 does not pull out from the rolled condom 43 without unrolling the condom in the process. Different shaped ends 32 may be more appropriate when the coefficient of friction between the unrolling strap 31 and the rolled condom 43 is less than the force required to stretch the condom 43 over the girth of the penis 45. Thus, the unrolling strap 31 should not be able to disengage from the condom 43 until the condom 43 is unrolled as fully as possible onto the wearer's penis 45.

For ease of manufacturing, the separately formed unrolling straps 55, may be cut from a single piece of material before being heat sealed to the first or second strips 25, 24. A plurality of curved cuts may be made at points along the single longer strap of material. This produces unrolling straps 55 wherein the end 59 of the unrolling strap 55 that is rolled up in the condom is convex, while the other end 61 of the unrolling strap that is heat sealed (at point 60) to the first or second strip 25, 24 has a concave shape 61. Having a concave end inside the condom ensures that the strap 55 remains flush against the side of the condom whilst the condom is being rolled up over the end 61 of the strap 55.

The unrolling straps 31 may have one or more perforated lines 33 extending across the width of the strap. In the event that the condom 43 is not able to fully unroll due to the user's penis being shorter than the length of the fully-unrolled condom, the unrolling strap 31 can break at one of these perforated lines 33. Thus, when the strain applied to a perforated line 33 is greater than the resistance to further unrolling of the condom 43, the unrolling strap 31 will break at that perforated line 33. Preferably, the perforated lines 33 get sequentially weaker the nearer each line is to the end 32 of the unrolling strap 31. Thus, the unrolling strap 31 will preferentially break at the perforated line 33 nearest the unrolled section of the condom 43. One method to ensure the perforated lines 33 get weaker towards the end 32 of the unrolling strap 31 is to have the width of the unrolling strap 31 taper towards the end 32 (not including the shaped end 32).

In the finished product, the sealed sleeve 23 forms the wrapper 23 that encloses the strip mechanism 50 and the rolled up condom 43. The mid-points 36 of the first strip 24 and the second strip 25 are respectively sealed to the sleeve 23 at the heat seals 22.

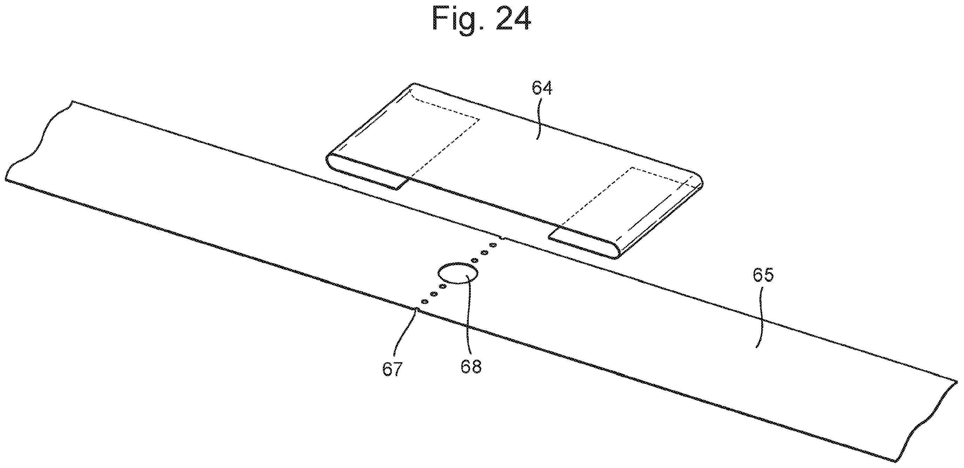

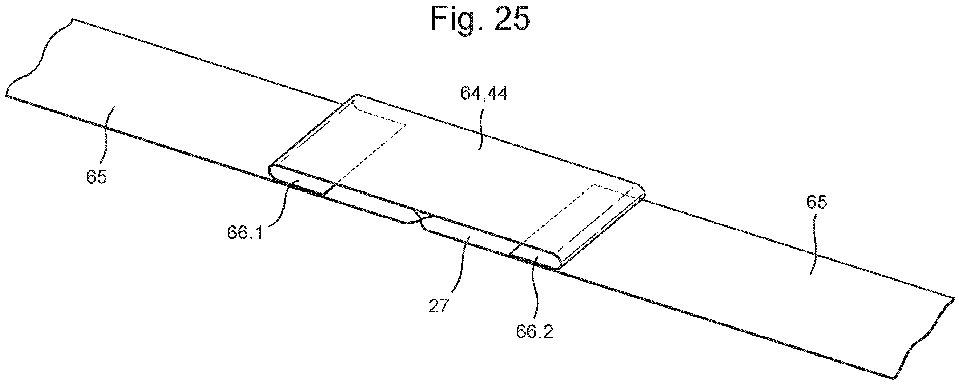

In an alternative arrangement, as shown in Figures 24-26, the condom holder may be formed from a longer first strip 65 and a shorter second strip 64. The first strip 65 has a hole 68 at a point distant from either end of the strip, preferably the hole is located approximately in the middle of the first strip 65 in terms of both the length and the width of the strip 65. Either side of the hole 68, extending across the width of the first strip 65, are perforations 67. The hole 38 is sized for receiving the tip 39 of the condom 43. The second strip 64 is heat sealed to the first strip 65 at two points 66.1, 66.2, one on either side of the hole 68. In this manner, the second strip forms the pocket 27 over the hole 68 and provides the shield region that covers the outer surface of the condom 43. The second strip 64 is extends between the two heat seal points 66.1, 66.2 such that it is neither slack nor tensioned, but lays flat against the first strip 65 when the first strip 65 is flat.

At either end of the first strip 65, there are unrolling straps 31, 55, either formed integrally with the first strip 65 or formed separately and heat sealed thereto. These unrolling straps 31, 55 are rolled up with the rolled up condom.

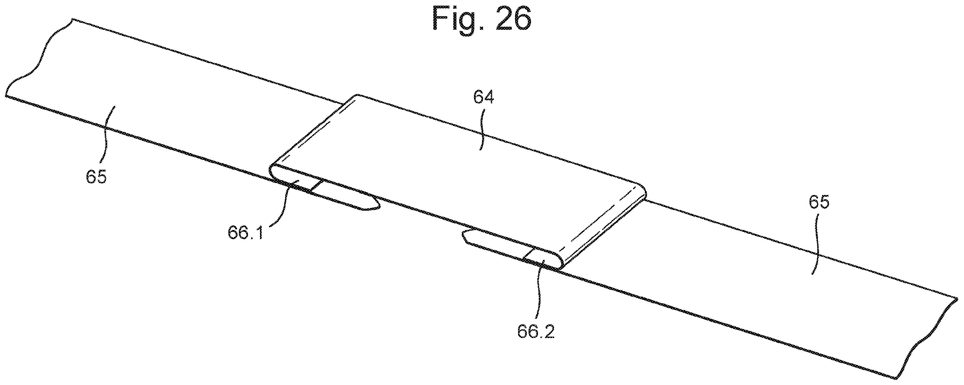

In use, the user pulls on either end of the first strip 65, either side of the hole 68, tensioning the first strip 65 and tensioning the second strip 64 via the heat seals 66.1, 66.2. Before the seals 66.1, 66.2 yield, the first strip 65 breaks apart along the line of perforations 67, as shown happening in Figure 26. At this point, the outer surface of the rolled-up condom is still shielded by the second strip 64. The unrolling straps 31, 55 are thus tensioned between the ends of the first strip being pulled in either directions away from the condom 43, and the straps 31, 55 cause the condom 43 to begin to unroll onto the penis 45.

The second strip 64 is folded underneath itself at each region 66.1, 66.2 where it is sealed to the first strip 65. This ensures that the heat seals yield gradually, not suddenly.

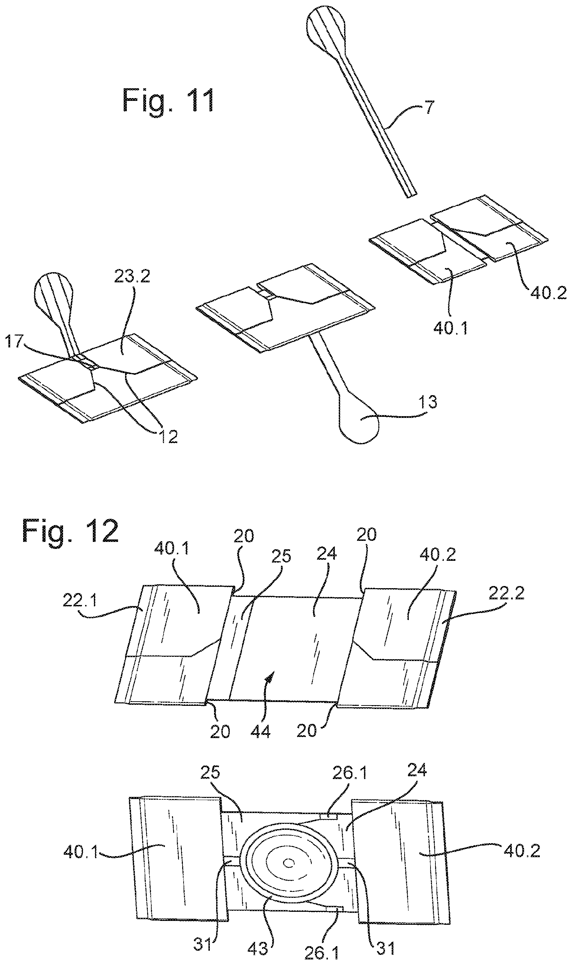

A method for unsealing the condom wrapper and applying the condom to a penis will now be described. Figure 11 shows the steps of unsealing the wrapper 23. On the left hand drawing, the user grips the pull tab 13 between thumb and forefinger and pulls up on the pull tab 13. The pull tab 13 attaches to a central tear strip 7 taking the form of a reinforcing strip that extends along the length of the sleeve blank 10 (that is formed, during manufacture, into the sleeve 23). The pull tab 13 advantageously has a textured surface that makes it easy to grip.

In the finished product, the central tear strip 7 is parallel to the heat seals 22 that seal the sleeve 23 to the strip mechanism 50. Parallel to, and on either side of, the central tear strip 7 there may optionally be another reinforcing strip 8, which serve to guide the removal of the central tear strip 7.

When the pull tab 13 is first pulled up by the user, it first tears the perforations 12 that prevent accidental opening of the wrapper by holding the pull tab 13 flat against the rest of the sleeve 23. The tearing of these perforations 12 may mimic the action and sound of undoing a zipper.

The central tear strip 7 is then unwound from the wrapper 23 until it is completely separated from the remainder of the wrapper 23 at which point the removed pull tab 13 and central tear strip 7 may be discarded. Thus, the wrapper portions 40.1 40.2 of the sleeve 23 that were previously joined together by the central tear strip 7 are no longer joined and the wrapper/sleeve 23 is now "unsealed". One portion 40.1 is heat sealed to the first strip 25 at a seal 22.1 and the other portion 40.2 is sealed to the second strip 24 at another seal 22.2.

Figure 12 shows an open sleeve 23. The sleeve 23 is now "open" and has been split into two wrapper portions 40.1 40.2, which at this point will function as pull tabs. The two wrapper portions 40.1, 40.2 may be easily gripped between the user's thumb and first finger.

The wrapper portions 40.1 and 40.2 are now pulled apart from one another in order to expose the condom and its holder. As the two strips 24, 25 are flexible strips folded in a 'Z' shaped cross-section 42 then they offer minimal resistance to being unfolded into a substantially straight line. Once the Z-shaped section 42 of each strip has been extended into an unfolded, straight configuration then further separation of the two wrapper portions 40.1 40.2 of the wrapper is resisted by the heat seals 26.1 and 26.2.

At this stage, the user can locate the condom 43 correctly in place without the possibility of accidentally beginning to unroll the condom through applying an unrolling force to the unrolling straps 31. It is not possible to place the condom upside down, since the tip of the condom 43 is pinched in the closable pocket 27 and parts of the strips 25, 24 form a shield region that fully covers the outside surface of the condom 43. Once the condom 43 is properly located on the tip of the penis, the user pulls the wrapper portions 40.1, 40.2 further apart to break the heat seals 26.1, 26.2 that bind the strips 25, 24 (and hence bind the wrapper portions 40.1, 40.2) together.

The first and second strips 25, 24 may be made of a clear material to allow the user to easily see where the condom 43 is relative to the tip of the penis 45. Alternatively or additionally, the user is able to use the step 20 created by the difference in width between each portion 40.1, 40.2 of the sleeve 23 and the exposed sections of the first 25 and second 24 strips, respectively, as a guide to assist in locating the condom correctly against the end of the penis 45. When the Z-shaped section has been made straight, the exposed top of the second strip 24 provides a space 44 that may be printed with a diagram of a random sex position, a marketing message, or other branding that was not visible to the user prior to opening the wrapper 23.

The space 44 may be printed with brand logos, barcodes, or QR codes (potentially linking to an adult website or to the manufacturer's website). Alternatively or additionally, space 44 may have printed holograms or lenticular prints illustrating sex positions, which images may appear to "move" when the user shifts the perspective by partially rotating the wrapper.

Once the user has correctly located the inner surface of the condom 43 against the penis 45, the user pulls the wrapper portions 40.1 and 40.2 further apart to break the heat seals 26.1, 26.2, while the tip 39 of the condom still remains trapped in the pocket 27 formed between the two strips 25, 24. The outer surface 38 of the condom remains fully covered by the strips 25, 24. Only the inner surface of the condom 43 is uncovered such that it may be placed against a penis 45. This protects the outer surface 28 of the condom 43 from contamination, particularly contamination resulting from attempting to put the condom 43 on from the wrong side before the user flips the condom 43 over to rectify their error.

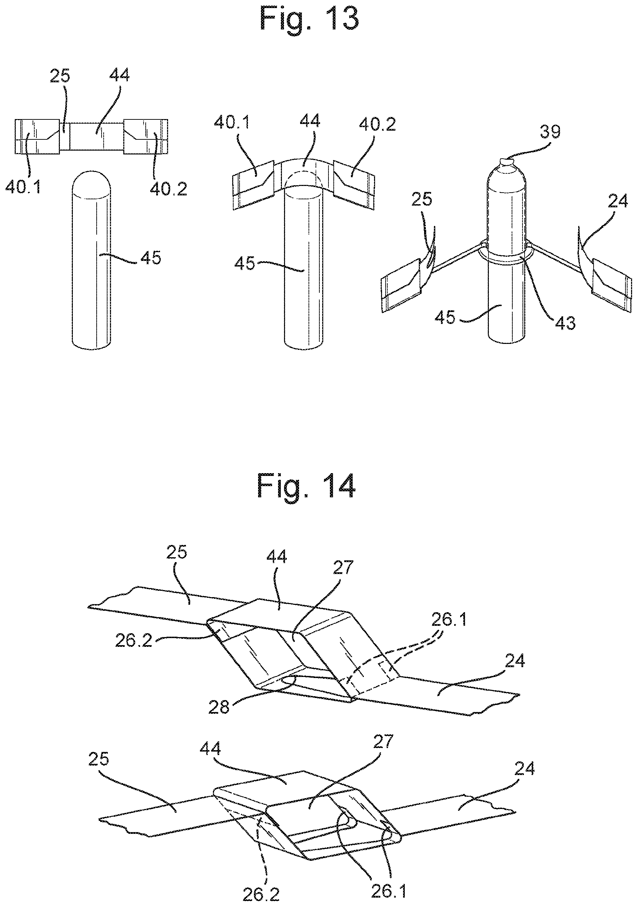

Figure 13 shows three steps in applying the condom 43 to a penis 45.

Step 1 (left hand drawing): The sleeve 23 has been opened, as described above with reference to Figure 11, exposing the strips 25, 24 for the first time. The Z-shaped sections 42 of the first and second strips 25, 24 have been pulled straight, exposing the shield area 44 of the second strip 24.

Step 2 (middle drawing): The user has located the condom 43 correctly over the end of the penis 45, and the user begins to increase the pulling force being applied to the two wrapper portions 40.1, 40.2 of the sleeve 23. This begins to break the heat seals 26 holding the first and second strips together. Simultaneously the unrolling straps 31 begin to pull out from the condom 43, thus starting to unroll the condom onto the penis 45, causing the condom 43, to be stretched in width, substantially sealing it around the head of the penis 45.

Step 3 (right band drawing): The heat seals 26 that bound the two strips 25, 24, together have pulled apart. Without these seals 26 acting as a brake, the pulling force on the wrapper portions 40.1, 40.2 allow the unrolling straps 31 to pull out from the condom 43 more easily, thus unrolling the condom down the remaining length of the penis 45.

The tip 39 of condom 43 is only released from the pocket 27 from Step 3 onwards. By Step 2, the condom 43 is substantially sealed around the head of the penis 45, which prevents air from entering the tip 39 of the condom when it is released from the pocket 27.

The 'V' section 28 of the first strip 25 allows the first strip 25 to be pulled away from the tip 39 of the condom 43 without fouling the tip 39. When the strips 25, 24 are being pulled apart and the heat seals 26.1, 26.2 are breaking (or broken), the shield surface 44 of the second strip 24 travels over the top of the condom 43 in one direction, while the 'arms' of the V-shaped section 28 of the first strip 25 travel either side of the condom 43 in the opposite direction. In this process, the second strip 24 is travelling in a direction that does not stretch the tip 39 of the condom 43, but instead friction between the second strip 24 and the tip 39 will tend to pull the tip 39 free from the V-shaped section 28 of the first strip 25.

Step 4 (not shown) involves the two unrolling straps 31 coming clear from the condom 43, either by having fully unrolled the condom 43 onto the user's penis or by having broken at the aforementioned perforations 33 in the case where unrolling of the condom 43 could not be completed. At no point during these steps has there been any skin contact with the condom 43 other than directly onto the penis 45. In particular, the condom need not be handled by the user: the user instead grips other parts of the wrapping and the condom holder.

Figure 9 shows one option for the ends 32 of the unrolling straps 31 and the changes in the unrolling straps' width. As described above, the strips 25, 24 are required to vary in width along their length. The reason for this is that at the region where the two strips 25, 24 overlap and are joined together at the seals 26.1, 26.2 the upper surface of the second strip 24 acts as a shield 44 to prevent accidental contact between the penis 45 and the outside of the condom 43 in the event that the condom 43 is presented 'wrong side up' to the penis 45. At this shield region 44, the second strip 24 must be at least as wide as the diameter of the rolled condom 43.

The other end of each strip 24, 25 comprises the unrolling straps 31 that are used to apply an unrolling force to the rolled condom 43. Thus, the width of the unrolling straps 31 is made smaller so that as the unrolling straps 31 are unrolling the condom 43, they do not impede the stretching of the condom 43 so as to give a tight and secure fit on the penis 45.

The shield region 44 of the second strip 24 has a constant width 30 from the heat seal 26.2 to the first strip 25, all the way up to and including the heat seal 22 to the sleeve 23. Similarly, the section of the first strip 25 extending from the heat seal 26.1 with the second strip 24, up to and including the heat seal 22 to the sleeve 23 (which region includes the V-shaped section 28) has a constant width 30 equal to the width of the second strip 24 in the shield section 24. Having the strips 25, 24 of equal width in these respective regions ensures that the pulling force that is applied to the wrapper portions 40.1, 40.2 to separate the two strips 25, 24 is transferred evenly to the heat seals 26.

The change in width between these wider regions of the first and second strips 25, 24 and the unrolling straps 31 may occur as either a taper or a step change 29 at any point between the heat seal 22 with the sleeve 23 and the start of the rolled condom 43.

In the event that the condom has unrolled as much as possible, and the unrolling straps 31 have not pulled out from the remainder of the rolled condom 43 but have broken at a perforated section 33, then the section of the unrolling strap 31 that remains protruding from the rolled-up remainder of condom 43 can be made to roll back by making the unrolling straps 31 from a material with shape memory properties.

Figure 16 shows the folding of the first strip 25. This drawing shows generally how each of the first and second strips 25, 24 is folded (as noted above, the main difference between the first 25 and second strips 24 is the V-shaped section 28). Each strip is folded back on itself at a mid-point 36. One end region of each folded strip, once joined with the other strip (as shown in Figures 14 and 15), will create a pocket 27 to secure the tip 39 of the condom 43. The other end of the folded strip comprises the unrolling strap 31 which will be rolled together with the condom 43. The mid-point 36 is the portion of each strip 24, 25 that is heat-sealed at positions 22.1, 22.2, respectively, to the sleeve 23. The variation in width of each strip (at position 29) may occur at any point between the mid-point 36 and the point where the unrolling strap 31 first meets the rolled condom 43.

Figure 15 illustrates how the tip 39 of the condom 43 is bent over and flattened between the strips 25, 24. Figure 15 shows how the two strips 25, 24, which have been sealed together, may be folded back to open the pocket 27 for receiving the condom tip 39. During manufacturing, after being sealed together at points 26.1, 26.2, the two strips 25, 24 are parted (such as by using a vacuum cup on the outer surface of each strip, or by another suitable method). The unrolled condom 43 is aligned with the V-shaped portion 28 of the first strip 25, with the tip of the condom extending through the V-shaped portion 28 into the pocket 27 between the two strips 25, 24. The unrolling straps 31 are rolled up with the condom 43 and then the tip 39 is pinched shut in the pocket 27 by pulling on the strips 25, 24. The second strip 24 is not brought straight downwards towards the first strip 25, but is instead brought down and across at an angle such that the tip 39 of the condom 43 is bent over to one side, pointing along the direction of the V of the V-shaped section 28, and the tip 39 lays flat against the two strips 25, 24.

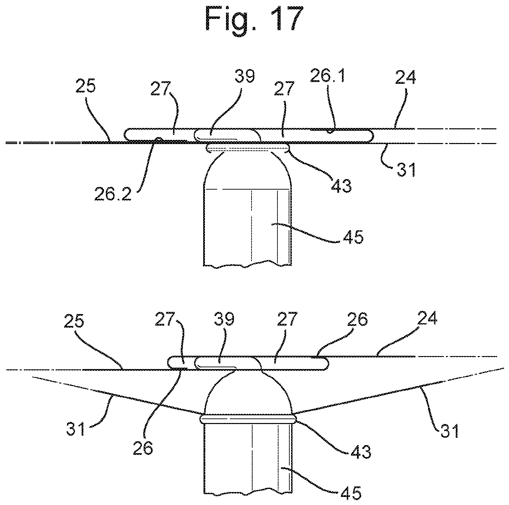

Figure 17 shows how the condom 43 begins to unroll over end of the penis 45 before the condom tip 39 is released, thus preventing air from being able to enter the tip 39 of the condom 43. This Figure shows the unrolling of the condom 43 in two stages. In step 1 of Figure 17 (upper drawing), the condom 43 has been positioned on the tip of the penis 45 but has not yet begun to unroll. Any air that may have previously entered the tip 39 of the condom 43 is forced out as the tip 39 is squeezed between the two strips 25, 24 when force is applied to the wrapper portions 40.1, 40.2 to begin the unrolling. The air is forced out of the tip 39 and is able to escape between the condom 43 and the penis 45 as, in step 1, there is either no contact or only a light contact force between the condom 43 and the penis 45 as at this point it has only just been located by the user into the correct position.

In step 2 of Figure 17 (lower drawing), the condom 43 is being unrolled onto the penis 45 by tension applied to the unrolling straps 31 as the wrapper portions 40.1, 40.2 are pulled apart. In step 2, the tip 39 is still pinched between the two strips 25, 24. The heat seals 26.1, 26.2 that connect the first 25 and second strips 24 are beginning to fail. By step 2, the condom 43 has started to unroll onto the penis 45, this causes the diameter of the condom 43 to be stretched (within design limits) by the unrolling of the straps 31. At this stage, the user may reposition the condom 43, even after the condom 43 has slightly unrolled. Even if the condom 43 is repositioned, the tip is still being pinched between the strips 24, 25 and the outer surface 38 is still being shielded by the shield region 44. Once the first and second strips 25, 24 have been fully pulled apart it is no longer possible for air to enter into the tip 39 of the condom 43 because, by this stage, the condom 43 is unrolled enough to provide an airtight seal between the condom 43 and the penis 45.

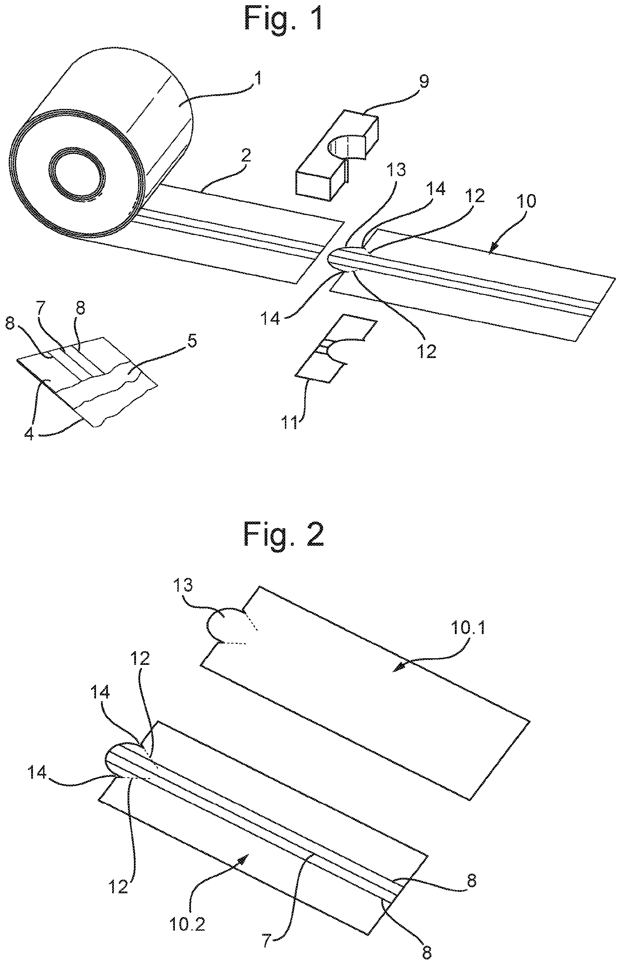

The manufacturing steps for the finished product are shown in sequence in Figures 1-4. Figure 1 shows a roll 1 of material 2 for forming the sleeve 23 and a die cutter 9 for cutting the roll 1.

The material 2 for forming the sleeve 23 is a flat, flexible material that may be made by laminating together several layers of other flat flexible materials 3. The material chosen for the outer layers 4 of the material 2 must be heat sealable at least to itself.

Condoms are classified as a type II medical device (as per ISO 4074). One layer 5 of the multi-material laminate material 2 used to make the sleeve 23 may be aluminium 5, which assists in making the sleeve 23 opaque to light. An opaque outer sleeve 23 is a key requirement of the ISO standard intended to reduce micro-bacterial growth and prolong the shelf life of the product.

At least one and up to three parallel tear strips 7, 8 may be laminated to the material 2. These provide a directionally reinforced section with the tensile strength required to allow the easy opening of the sleeve 23. The central tear strip 7 is essential. Two other tear strips 8 may be disposed one on either side of the central tear strip 7, that prevent the condom wrapper product from distorting when the central tear strip 7 is opened. That is, to prevent the line of tearing from deviating from the central part of the sleeve 23.

The die cutter 9 may be used to cut the roll 1 into short predetermined lengths, "blanks" 10, each blank 10 being sufficient to make one sleeve 23. The die cutter 9 may cut the outline of the pull tab 13 and may also crimp the material in this region so that the pull tab 13 has a textured gripping surface for easy gripping.

The die cutting process will create a small amount of waste material 11 as the sleeve blanks are not shaped the same at both ends.

In addition to separating the wrapper blank 10 from the roll of material 1, the die cutter 9 (or another cutter not shown, or another process such as a laser cutter) will cut perforations 12 into the material 2. In the arrangement shown in Figures 1, 2, 3, and 6, these perforations 12 are shown as small holes that complete the outline of the pull tab 13 that is used by the user to open the sleeve 23. Prior to breaking, the perforations 12 keep the pull tab 13 aligned flat with the rest of the sleeve 23, preventing accidental opening. In an example wrapper containing three tear strips 7, 8, the perforations 12 must "cut" across the outer two tear strips 8 such that, when the perforations 12 are broken, the pulling force on the pull tab 13 is transferred primarily to the central tear strip 7. Notches 14 may be formed by the die cutter 9, to act as a lead when the user lifts the pull tab 13 and begins to tear through the perforations 12.

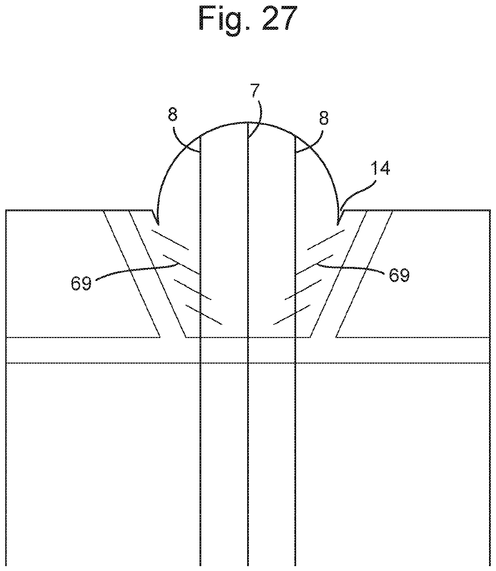

In an alternative arrangement, as shown in Figure 27, the perforations 12 have an elongate shape 69 and form a "herringbone" pattern wherein at least one of the elongate perforations 69 cuts across one of the tear strips 8. This shape of perforations can help to ensure a clean cut and aids removal of the central tear strip 7 to open the finished wrapper.

Figure 2 shows views from both sides of a sleeve blank 10. The branding, logo, promotional information and/or user instructions may be printed on the surface 10.1 that becomes the outer surface of the sleeve 23. The expiry date and/or batch/lot numbers may be printed at this stage or added later. One surface 10.2 of the sleeve blank 10 will become the inner surface when the sleeve 23 is formed. This surface 10.2 may remain unprinted as it is not easily visible at any stage of opening the finished product.

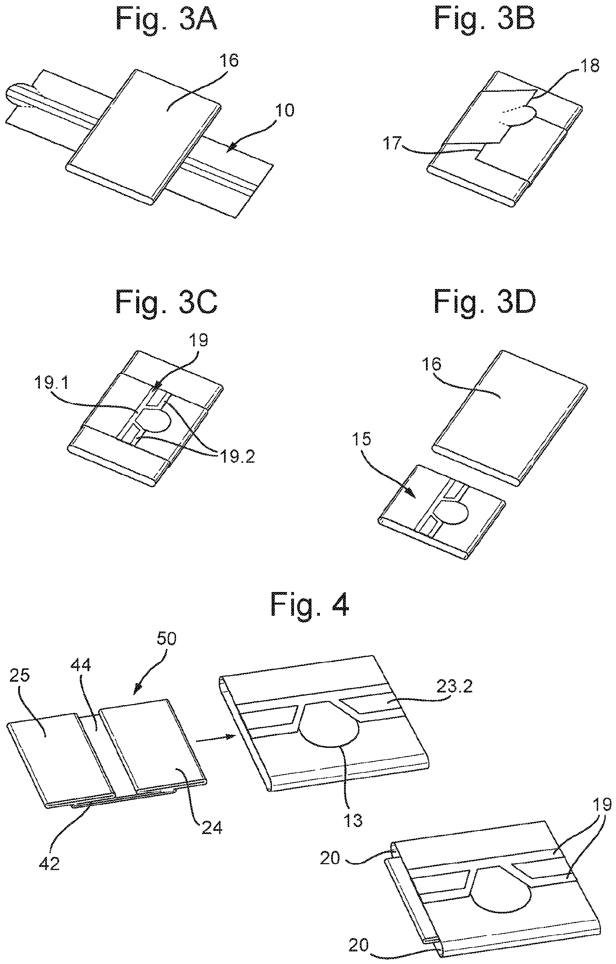

Figures 3A-D show the steps of folding a sleeve blank 10 around a former 16 to turn the sleeve blank 10 into a sleeve 23. Figure 3A shows the first step wherein the former 16 is placed on the blank 10. In Figure 3B, the sleeve blank 10 is wrapped around the former 16 so that the short straight edge 17 of the sleeve blank 10 is located between the former 16 and the end 18 of the sleeve blank 10 having the pull tab 13. The end 18 having the pull tab 13 will overlap the other edge 17 by a short distance.

A press, such as a heat press, an ultrasonic welder, or similar, is used to form a heat seal 19.1, 19.2 (together 19) that seals the two edges 17, 18 together. The shape of the heat seal 19, as shown in Figure 3C, may be broadly in the shape of a stylised capital letter 'K'. The straight upright portion of the 'K' 19.1 will eventually form part of the wrapper seal. The remaining portions of the 'K' 19.2 are used to hold down the section of the sleeve 23 that overlaps itself. This ensures that the pull tab 13 remains aligned flat to the finished sleeve 23. The perforations 12 are located adjacent to the heat seal 19, ensuring that the pull tab 13 is easy to lift. After heat sealing the two ends 17, 18 together the sleeve 23 is removed from the former 16 by sliding the sleeve 23 off, as shown in Figure 3D. At this point, the sleeve 23 is toroidal.

Figure 4 shows the step of inserting the rolled condom 43 and the strip mechanism 50 into the sleeve 23 through one of the open sides of the toroidal sleeve 23. Once the sleeve 23 has been formed, the wrapper is loaded with the strip mechanism 50 and a rolled condom 43 by sliding the strip mechanism 50 and the condom 43, which has been pre rolled into the folded strips (shown in other drawings and described above), through one of the open sides of the sleeve 23. The width of the strip mechanism is slightly less that the width of the open sides of the sleeve 23. Thus, there are small gaps 20 between the sleeve 23 and either end of the strips (across their width).

Figure 5 shows two additional heat seal processes required for sealing the open sides of the sleeve 23 to form the wrapper 23. The heat seal 19 that formed the sleeve blank 10 into the sleeve 23 must be performed before the second seal 22 that affixes the strip mechanism 50 to the sleeve 23. When the strip mechanism and condom have been inserted into the sleeve 23, the second heat sealing may be performed. Two straight seals 22.1, 22.2 are made simultaneously, enclosing the condom 43 and strip mechanism 50 inside the sleeve 23 by sealing the two open edges of the sleeve 23 to the strips 25, 24 of the strip mechanism 50. As stated previously, these heat seals 22.1, 22.2 may either be made by a heat press, an ultrasonic welder or any similar seal that will create a permanent thermal seal.

A condom wrapper as described above can offer the following advantages
- The outer surface of the condom is always concealed by the wrapper until the condom actually begins to unroll onto the penis. This way there is no chance of the user making the common mistake of incorrectly orientating the condom, touching the outer surface against the tip of the penis first and then 'flipping' it over to the correct side before unrolling it.
- The tip of the condom is always pinched closed by the wrapper until the condom begins to actually unroll onto the penis. This eliminates the possibility of trapping air in the reservoir tip of the condom, leaving no space to contain the semen.
- Since there is no need to remove the condom from the wrapper there is no chance of damaging the condom with fingernails or jewellery etc. Additionally, there is no possibility of transferring any bodily fluids or non-water-based lubricants (which may damage latex condoms) to the outer surface of the condom.
- By ensuring that the condom is unrolled to the fullest extent possible the chances of the condom slipping off during intercourse are reduced.
- By unrolling the condom in this manner, it is possible to avoid the possibility of accidently transferring lubricant from the condom onto the penis, as can easily occur when unrolling the condom by hand. Therefore avoiding a situation where the lubricant can come to be between the condom and the penis making it easier for the condom to slip off whilst in use.

## Claims

1. A holder for a condom (43) having a tip (39), the holder comprising
a closable pocket (27) for pinching the tip (39) of the condom (43) when the pocket (27) is closed; and
a shield region forming part of the closable pocket (27) for covering the outer surface of the condom (43) when the condom (43) is rolled up and the holder is in use;
wherein the holder is arranged to continue to pinch the tip (39) and shield the outer surface of the condom (43) until after the user has started to unroll the condom (43) and the condom (43) has therefore had the opportunity to seal around the head of the penis.

2. A holder as claimed in claim 1, wherein the holder comprises
a first strip (25) having a first end (26.1) and a second end; and
a second strip (24) having a first end (26.2) and a second end; wherein
the first end (26.1) of the first strip is joined to the second strip at a point distant from the first end of the second strip, and
the first end (26.2) of the second strip is joined to the first strip at a point distant from the first end of the first strip,
whereby the closable pocket (27) is formed between the first strip and the second strip.

3. A holder as claimed in claim 2, wherein the closable pocket is formed by a loop of material made from a part of the first strip extending from the first end of the first strip to the point at which the first end of the second strip joins the first strip and from a part of the second strip extending from the first end of the second strip to the point at which the first end of the first strip joins the second strip.

4. A holder as claimed in claim 2 or 3, wherein the first strip has an opening (28) adjacent to the closable pocket for receiving the tip (39) of the condom, and wherein the strips are arranged to pinch the tip in the closed pocket when the tip is passed through the opening.

5. A holder as claimed in claim 1, wherein the holder comprises
a first strip (65) having a first end and a second end; and
a second strip (64) having a first end (66.1) and a second end (66.2); wherein
the first end of the second strip is sealed to the first strip at a first point distant from both ends of the first strip, and
the second end of the second strip is sealed to the first strip at a second point distant from both ends of the first strip, such that the closable pocket is formed between the second strip and first strip; preferably comprising:
a hole (68) in the first strip located between the first and second points and opening into the closeable pocket; and
a line of perforations (69) in the first strip, said line extending across the width of the first strip and intersecting the hole.

6. A holder as claimed in any of claims 2 to 5, wherein the joins between the strips use adhesive elements or heat sealing.

7. A holder as claimed in any of claims 2 to 6, wherein the second strip is arranged to provide the shield region for completely covering the outer surface of the condom when the condom is, in use, mounted in the holder with the tip in the closable pocket.

8. A holder as claimed in any of claims 2 to 7, wherein a portion of one or both strips of the holder is folded into a Z-shaped configuration; preferably wherein the Z-shaped portion (42) extends away from the closable pocket and shield region.

9. A holder as claimed in any of claims 2 to 8, wherein a free end of the first or the second strip includes an unrolling strap (31;55) that is, in use, rolled up along the outer surface of the rolled condom, wherein the unrolling strap is for unrolling the condom; preferably wherein a second free end of the first or second strip comprises an unrolling strap (31) that is rolled up with the condom on the opposite side of the condom from the first strip's unrolling strap.

10. A holder as claimed in any of claims 2 to 8, comprising a first unrolling strap (31;55) sealed to one of the first or second strip in a region of said strip adjacent the closable pocket; wherein the first unrolling strap, in use, is rolled up along the outer surface of the rolled condom, and wherein the first unrolling strap is for unrolling the condom; and preferably wherein a second unrolling strap (31;55) is sealed to the first or second strip at a region on the opposite side of the closable pocket from the first unrolling strap; wherein the first unrolling strap, in use, is rolled up along the outer surface of the rolled condom, and wherein the second unrolling strap is for unrolling the condom.

11. A holder as claimed in any of claims 9 or 10, wherein the unrolling straps are arranged to release from the condom or to break in order to fully disconnect the condom from the holder after the condom has been unrolled.

12. A packaging system for a condom, the packaging system comprising:
a holder as claimed in any preceding claim, and
an outer wrapper (23) that encloses both the holder and a rolled up condom (43).

13. A method of packaging a condom (43) having a tip (39), in a holder according to any of the preceding claims, the method comprising:
pinching the tip in the closable pocket (27) of the holder; and
completely covering the outer surface of the rolled condom with the shield region of the holder; and
arranging the holder so that it will continue to pinch the tip and shield the outer surface of the condom until after the user has started to unroll the condom and the condom has therefore had the opportunity to seal around the head of the penis.

14. A method as claimed in claim 13, wherein the holder comprises a first strip (25) and a second strip (24) and the method includes:
joining a first end (26.1) of the first strip to the second strip at a point distant from a first end (26.2) of the second strip, and
joining the first end (26.2) of the second strip to the first strip at a point distant from the first end of the first strip to form the closable pocket between the first strip and the second strip.

15. A method as claimed in any of claims 13 to 14 comprising:
inserting the holder into a wrapper blank (10), and then
sealing the wrapper blank (10) about the holder simultaneously with coupling two portions of the wrapper to the strips.

## Patentansprüche

1. Halter für ein Kondom (43) mit einer Spitze (39), wobei der Halter umfasst
eine verschließbare Tasche (27) zum Einklemmen der Spitze (39) des Kondoms (43), wenn die Tasche (27) geschlossen ist; und
einen Abschirmbereich, der Teil der verschließbaren Tasche (27) bildet, um die Außenfläche des Kondoms (43) abzudecken, wenn das Kondom (43) aufgerollt wird und der Halter in Verwendung ist;
wobei der Halter so angeordnet ist, dass er weiter die Spitze (39) einklemmt und die Außenfläche des Kondoms (43) abschirmt, bis der Benutzer mit dem Abrollen des Kondoms (43) begonnen hat und das Kondom (43) daher die Möglichkeit hat, sich dicht um den Peniskopf zu legen.

2. Halter nach Anspruch 1, wobei der Halter umfasst
einen ersten Streifen (25) mit einem ersten Ende (26.1) und einem zweiten Ende; und
einen zweiten Streifen (24) mit einem ersten Ende (26.2) und einem zweiten Ende; wobei
das erste Ende (26.1) des ersten Streifens mit dem zweiten Streifen an einem Punkt verbunden ist, der vom ersten Ende des zweiten Streifens entfernt ist, und
das erste Ende (26.2) des zweiten Streifens mit dem ersten Streifen an einem Punkt verbunden ist, der vom ersten Ende des ersten Streifens entfernt ist,
wobei die verschließbare Tasche (27) zwischen dem ersten Streifen und dem zweiten Streifen ausgebildet ist.

3. Halter nach Anspruch 2, wobei die verschließbare Tasche durch eine Materialschleife gebildet ist, die aus einem Teil des ersten Streifens gebildet ist, der sich vom ersten Ende des ersten Streifens bis zu dem Punkt erstreckt, an dem das erste Ende des zweiten Streifens mit dem ersten Streifen verbunden ist, und von einem Teil des zweiten Streifens, der sich vom ersten Ende des zweiten Streifens bis zu dem Punkt erstreckt, an dem das erste Ende des ersten Streifens mit dem zweiten Streifen verbunden ist.

4. Halter nach Anspruch 2 oder 3, wobei der erste Streifen eine Öffnung (28) angrenzend an die verschließbare Tasche zur Aufnahme der Spitze (39) des Kondoms aufweist, und wobei die Streifen so angeordnet sind, dass sie die Spitze in der geschlossenen Tasche einklemmen, wenn die Spitze durch die Öffnung geführt wird.

5. Halter nach Anspruch 1, wobei der Halter umfasst
einen ersten Streifen (65) mit einem ersten Ende und einem zweiten Ende; und
einen zweiten Streifen (64) mit einem ersten Ende (66.1) und einem zweiten Ende (66.2); wobei
das erste Ende des zweiten Streifens mit dem ersten Streifen an einem ersten Punkt, der von beiden Enden des ersten Streifens entfernt ist, versiegelt ist, und
das zweite Ende des zweiten Streifens an einem zweiten Punkt, der von beiden Enden des ersten Streifens entfernt ist, mit dem ersten Streifen versiegelt ist, sodass die verschließbare Tasche zwischen dem zweiten Streifen und dem ersten Streifen gebildet wird; vorzugsweise umfassend:
ein Loch (68) in dem ersten Streifen, das zwischen dem ersten und dem zweiten Punkt angeordnet ist und sich in die verschließbare Tasche öffnet; und
eine Linie von Perforationen (69) in dem ersten Streifen, wobei sich die Linie über die Breite des ersten Streifens erstreckt und das Loch schneidet.

6. Halter nach einem der Ansprüche 2 bis 5, wobei die Verbindungen zwischen den Streifen Klebeelemente oder Heißsiegelung verwenden.

7. Halter nach einem der Ansprüche 2 bis 6, wobei der zweite Streifen so angeordnet ist, dass er den Abschirmbereich zum vollständigen Abdecken der Außenfläche des Kondoms bereitstellt, wenn das Kondom bei Verwendung in dem Halter mit der Spitze in der verschließbaren Tasche angebracht ist.

8. Halter nach einem der Ansprüche 2 bis 7, wobei ein Abschnitt eines oder beider Streifen des Halters zu einer Z-förmigen Konfiguration gefaltet ist; vorzugsweise wobei sich der Z-förmige Abschnitt (42) von dem verschließbaren Taschen- und Abschirmbereich weg erstreckt.

9. Halter nach einem der Ansprüche 2 bis 8, wobei ein freies Ende des ersten oder des zweiten Streifens ein Abrollband (31;55) beinhaltet, das bei Verwendung entlang der Außenfläche des gerollten Kondoms aufgerollt wird, wobei das Abrollband zum Abrollen des Kondoms dient; vorzugsweise wobei ein zweites freies Ende des ersten oder zweiten Streifens ein Abrollband (31) umfasst, das mit dem Kondom auf der gegenüberliegenden Seite des Kondoms vom Abrollband des ersten Streifens aufgerollt wird.

10. Halter nach einem der Ansprüche 2 bis 8, umfassend ein erstes Abrollband (31;55), das mit einem der ersten oder zweiten Streifen in einem Bereich des Streifens benachbart zur verschließbaren Tasche versiegelt ist; wobei das erste Abrollband bei Verwendung entlang der Außenfläche des gerollten Kondoms aufgerollt ist, und wobei das erste Abrollband zum Abrollen des Kondoms dient; und vorzugsweise, wobei ein zweites Abrollband (31;55) an dem ersten oder zweiten Streifen an einem Bereich auf der gegenüberliegenden Seite der verschließbaren Tasche vom ersten Abrollband versiegelt ist; wobei das erste Abrollband bei Verwendung entlang der Außenfläche des gerollten Kondoms aufgerollt wird, und wobei das zweite Abrollband zum Abrollen des Kondoms dient.

11. Halter nach einem der Ansprüche 9 oder 10, wobei die Abrollbänder so angeordnet sind, dass sie sich vom Kondom lösen oder reißen, um das Kondom nach dem Abrollen des Kondoms vollständig vom Halter zu trennen.

12. Verpackungssystem für ein Kondom, wobei das Verpackungssystem umfasst:
einen Halter nach einem der vorstehenden Ansprüche, und
eine äußere Hülle (23), die sowohl den Halter als auch ein aufgerolltes Kondom (43) umschließt.

13. Verfahren zum Verpacken eines Kondoms (43) mit einer Spitze (39) in einem Halter nach einem der vorstehenden Ansprüche, wobei das Verfahren umfasst:
Einklemmen der Spitze in die verschließbare Tasche (27) des Halters; und
vollständiges Bedecken der Außenfläche des gerollten Kondoms mit dem Abschirmbereich des Halters; und
Anordnen des Halters, so dass er die Spitze weiter einklemmt und die Außenfläche des Kondoms abschirmt, bis der Benutzer mit dem Abrollen des Kondoms begonnen hat und das Kondom somit die Möglichkeit hatte, sich dicht um den Peniskopf zu legen.

14. Verfahren nach Anspruch 13, wobei der Halter einen ersten Streifen (25) und einen zweiten Streifen (24) umfasst und das Verfahren beinhaltet:
Verbinden eines ersten Endes (26.1) des ersten Streifens mit dem zweiten Streifen an einem Punkt, der von einem ersten Ende (26.2) des zweiten Streifens entfernt ist, und
Verbinden des ersten Endes (26.2) des zweiten Streifens mit dem ersten Streifen an einem Punkt, der vom ersten Ende des ersten Streifens entfernt ist, um die verschließbare Tasche zwischen dem ersten Streifen und dem zweiten Streifen zu bilden.

15. Verfahren nach einem der Ansprüche 13 bis 14, umfassend:
Einsetzen des Halters in einen Streifbandrohling (10) und dann
Versiegeln des Streifbandrohlings (10) um den Halter herum gleichzeitig mit dem Koppeln zweier Abschnitte des Streifbandes mit den Streifen.

## Revendications

1. Support pour un préservatif (43) ayant un bout (39), le support comprenant
une poche refermable (27) pour pincer le bout (39) du préservatif (43) lorsque la poche (27) est fermée ; et
une zone protectrice formant une partie de la poche refermable (27) pour couvrir la surface externe du préservatif (43) lorsque le préservatif (43) est enroulé et le support est en utilisation ;
dans lequel le support est agencé pour continuer à pincer le bout (39) et protéger la surface externe du préservatif (43) jusqu'à ce que l'utilisateur ait commencé à dérouler le préservatif (43) et que le préservatif (43) ait donc eu l'opportunité d'entourer le gland du pénis.

2. Support selon la revendication 1, dans lequel le support comprend
une première bande (25) ayant une première extrémité (26.1) et une seconde extrémité ; et
une seconde bande (24) ayant une première extrémité (26.2) et une seconde extrémité ; dans lequel
la première extrémité (26.1) de la première bande est jointe à la seconde bande au niveau d'un point distant de la première extrémité de la seconde bande, et
la première extrémité (26.2) de la seconde bande est jointe à la première bande au niveau d'un point distant de la première extrémité de la première bande,
moyennant quoi la poche refermable (27) est formée entre la première bande et la seconde bande.

3. Support selon la revendication 2, dans lequel la poche refermable est formée par une boucle de matériau réalisée à partir d'une partie de la première bande s'étendant à partir de la première extrémité de la première bande vers le point au niveau duquel la première extrémité de la seconde bande rejoint la première bande et à partir d'une partie de la seconde bande s'étendant à partir de la première extrémité de la seconde bande vers le point au niveau duquel la première extrémité de la première bande rejoint la seconde bande.

4. Support selon la revendication 2 ou 3, dans lequel la première bande a une ouverture (28) adjacente à la poche refermable pour recevoir le bout (39) du préservatif, et dans lequel les bandes sont agencées pour pincer le bout dans la poche fermée lorsque le bout est passé à travers l'ouverture.

5. Support selon la revendication 1, dans lequel le support comprend
une première bande (65) ayant une première extrémité et une seconde extrémité ; et
une seconde bande (64) ayant une première extrémité (66.1) et une seconde extrémité (66.2) ; dans lequel
la première extrémité de la seconde bande est scellée à la première bande au niveau d'un premier point distant des deux extrémités de la première bande, et
la seconde extrémité de la seconde bande est scellée à la première bande au niveau d'un second point distant des deux extrémités de la première bande, de telle sorte que la poche refermable est formée entre la seconde bande et la première bande ; de préférence comprenant :
un trou (68) dans la première bande situé entre les premier et second points et s'ouvrant dans la poche refermable ; et
une ligne de perforations (69) dans la première bande, ladite ligne s'étendant à travers la largeur de la première bande et croisant le trou.

6. Support selon l'une quelconque des revendications 2 à 5, dans lequel les jonctions entre les bandes utilisent des éléments adhésifs ou un thermoscellage.

7. Support selon l'une quelconque des revendications 2 à 6, dans lequel la seconde bande est agencée pour fournir la zone protectrice pour couvrir entièrement la surface externe du préservatif lorsque le préservatif est, en utilisation, monté dans le support avec le bout dans la poche refermable.

8. Support selon l'une quelconque des revendications 2 à 7, dans lequel une partie d'une ou des deux bandes du support est pliée dans une configuration en forme de Z ; de préférence dans lequel la partie en forme de Z (42) s'étend en s'éloignant de la poche refermable et de la zone protectrice.

9. Support selon l'une quelconque des revendications 2 à 8, dans lequel une extrémité libre de la première ou de la seconde bande inclut un lien de déroulement (31 ; 55) qui est, en utilisation, enroulé le long de la surface externe du préservatif enroulé, dans lequel le lien de déroulement est destiné à dérouler le préservatif ; de préférence dans lequel une seconde extrémité libre de la première ou de la seconde bande comprend un lien de déroulement (31) qui est enroulé avec le préservatif sur le côté opposé du préservatif par rapport au lien de déroulement de la première bande.

10. Support selon l'une quelconque des revendications 2 à 8, comprenant un lien de déroulement (31 ; 55) scellé à l'une de la première ou de la seconde bande dans une zone de ladite bande adjacente à la poche refermable ; dans lequel le premier lien de déroulement, en utilisation, est enroulé le long de la surface externe du préservatif enroulé, et dans lequel le premier lien de déroulement est destiné à dérouler le préservatif; et de préférence dans lequel un second lien de déroulement (31 ; 55) est scellé à la première ou à la seconde bande au niveau d'une zone sur le côté opposé de la poche refermable par rapport au premier lien de déroulement; dans lequel le premier lien de déroulement, en utilisation, est enroulé le long de la surface externe du préservatif enroulé, et dans lequel le second lien de déroulement est destiné à dérouler le préservatif.

11. Support selon l'une quelconque des revendications 9 ou 10, dans lequel les liens de déroulement sont agencés pour se libérer du préservatif ou pour se rompre afin de détacher complètement le préservatif du support après que le préservatif a été déroulé.

12. Système de conditionnement pour un préservatif, le système de conditionnement comprenant :
un support selon une quelconque revendication précédente, et
un emballage externe (23) qui renferme à la fois le support et un préservatif enroulé (43).

13. Procédé de conditionnement d'un préservatif (43) ayant un bout (39), dans un support selon l'une quelconque des revendications précédentes, le procédé comprenant:
le pincement du bout dans la poche refermable (27) du support ; et
le fait de couvrir complètement la surface externe du préservatif enroulé avec la zone protectrice du support ; et
l'agencement du support de sorte qu'il continuera à pincer le bout et à protéger la surface externe du préservatif jusqu'à ce que l'utilisateur ait commencé à dérouler le préservatif et le préservatif ait ainsi eu l'opportunité d'entourer le gland du pénis.

14. Procédé selon la revendication 13, dans lequel le support comprend une première bande (25) et une seconde bande (24) et le procédé inclut :
la jonction d'une première extrémité (26.1) de la première bande à la seconde bande au niveau d'un point distant d'une première extrémité (26.2) de la seconde bande, et
la jonction de la première extrémité (26.2) de la seconde bande à la première bande au niveau d'un point distant de la première extrémité de la première bande pour former la poche refermable entre la première bande et la seconde bande.

15. Procédé selon l'une quelconque des revendications 13 à 14 comprenant :
l'insertion du support dans une découpe d'emballage (10), et ensuite
le scellement de la découpe d'emballage (10) autour du support simultanément au couplage de deux parties de l'emballage avec les bandes.
